# FASCICULE DE BREVET EUROPEEN

(11) **EP 2 531 215 B1**
(45) Date de publication et mention de la délivrance du brevet: **10.06.2015**
(21) Numéro de dépôt: 11705018.7
(22) Date de dépôt: 14.01.2011
(51) Int. Cl.: A61K 39/39, A61K 39/012

(54) **UTILISATION D'UNE HUILE MINERALE SPECIFIQUE POUR LA FABRICATION D'UN NOUVEAU ADJUVANT**
VERWENDUNG EINES SPEZIFISCHEN MINERALÖLS ZUR HERSTELLUNG EINES NEUEN HILFSSTOFFS
USE OF A SPECIFIC MINERAL OIL FOR THE PRODUCTION OF A NOVEL ADJUVANT

(30) Priorité: 01.02.2010 FR 1050668
(43) Date de publication de la demande: 12.12.2012
(73) Titulaire: Societe D'Exploitation De Produits Pour Les Industries Chimiques Seppic, 75007 Paris (FR)
(72) Inventeur: DUPUIS, Laurent, F-51100 Reims (FR); GAUCHERON, Jérôme, F-81100 Castres (FR); BERTRAND, François, 75014 Paris (FR)
(74) Mandataire: Grout de Beaufort, François-Xavier
(86) Numéro de dépôt international: PCT/FR2011/050067
(87) Numéro de publication internationale: WO 2011/092412

(56) Documents cités:
- WO-A2-2005/009462
- WO-A2-2006/081826
- Anonymous: "Marcol 52", Exxon Mobil , 2007, pages 1-3, XP002599991, Extrait de l'Internet: URL:http://www.exxonmobil.com/UK-English/S pecialties/PDS/glxxenspcemmarcol_52.pdf [extrait le 2010-09-09]
- Anonymous: "Marcol 82", Exxon Mobil , 2007, pages 1-3, XP002599992, Extrait de l'Internet: URL:http://www.exxonmobil.com/UK-English/S pecialties/PDS/glxxenspcemmarcol_82.pdf [extrait le 2010-09-09]
- DING XICHENG ET AL: "Protective immunity against Eimeria acervulina following in ovo immunization with a recombinant subunit vaccine and cytokine genes", INFECTION AND IMMUNITY, vol. 72, no. 12, décembre 2004 (2004-12), pages 6939-6944, XP002600560, ISSN: 0019-9567
- DUPUIS L ET AL: "SEPPIC vaccine adjuvants for poultry.", ANNALS OF THE NEW YORK ACADEMY OF SCIENCES, vol. 1081, octobre 2006 (2006-10), pages 202-205, XP002599990, ISSN: 0077-8923
- PIRETTI M V ET AL: "INVESTIGATION OF THE HYDRO CARBONS FOUND IN THE TISSUES OF CHICKENS INJECTED WITH INACTIVATED OIL ADJUVANT VACCINE", ZEITSCHRIFT FUER LEBENSMITTEL-UNTERSUCHUNG UND -FORSCHUNG, vol. 175, no. 4, 1982, pages 245-248, XP002599989, ISSN: 0044-3026
- DUNCAN E S STEWART-TULL: "The Use of Adjuvants in Experimental Vaccines. II. Water-in-Oil Emulsions: Freund s Complete and lncomplete Adjuvants", METHODS IN MOLECULAR MEDICINE, vol. 4, 1 janvier 2003 (2003-01-01), pages 141-145, XP001525280, HUMANA PRESS, TOTOWA, NJ, US ISSN: 1543-1894, DOI: 10.1385/0-89603-334-1:141
- AUCOUTURIER JEROME ET AL: "Montanide ISA 720 and 51: a new generation of water in oil emulsions as adjuvants for human vaccines", EXPERT REVIEW OF VACCINES, vol. 1, no. 1, 1 juin 2002 (2002-06-01), pages 111-118, XP002262967, FUTURE DRUGS, LONDON, GB ISSN: 1476-0584, DOI: 10.1586/14760584.1.1.111
- KURODA YOSHIKI ET AL: "Distinctive patterns of autoimmune response induced by different types of mineral oil", TOXICOLOGICAL SCIENCES, vol. 78, no. 2, avril 2004 (2004-04), pages 222-228, XP002599988, ISSN: 1096-6080
- STEWART-TULL DUNCAN E S: "Freund's complete and incomplete adjuvants, preparation, and quality control standards for experimental laboratory animals use", METHODS IN MOLECULAR BIOLOGY, HUMANA PRESS INC, NJ, US, vol. 626, 1 janvier 2010 (2010-01-01), pages 59-72, XP001525277, ISSN: 1064-3745, DOI: DOI:10.1007/978-1-60761-585-9_5
- ISEKI KANAKO ET AL: "Evaluation of a new oil adjuvant for use in peptide-based cancer vaccination.", CANCER SCIENCE OCT 2010 LNKD- DOI:10.1111/J.1349-7006.2010.01653.X PUBMED:20678155, vol. 101, no. 10, octobre 2010 (2010-10), pages 2110-2114, XP002631708, ISSN: 1349-7006

## Description

La présente invention concerne de nouveaux adjuvants destinés à la préparation de compositions vaccinales, des compositions vaccinales comprenant lesdits adjuvants, ainsi que l'utilisation d'une huile minérale spécifique pour la fabrication de ce dit adjuvant.

Le développement de vaccins inactivés ou contenant des antigènes purifiés est de plus en plus important car il permet d'éviter au maximum les effets secondaires indésirables chez les sujets vaccinés pendant et après la vaccination. Cependant l'amélioration de la qualité des antigènes se fait au détriment de leur immunogénicité. C'est pour cette raison qu'ils sont associés à des adjuvants permettant d'augmenter la réponse immunitaire chez les sujets vaccinés.

Ces adjuvants sont de natures diverses. Ils peuvent par exemple consister en des liposomes, des émulsions comprenant au moins une phase huile et au moins une phase aqueuse, du type adjuvants dits de Freund ou, de manière plus courante en des sels minéraux insolubles dans l'eau. Parmi les sels minéraux utilisés comme adjuvants de compositions vaccinales, on peut citer par exemple l'hydroxyde d'aluminium, le nitrate de cérium, le sulfate de zinc, l'hydroxyde de fer colloïdal ou le chlorure de calcium. L'hydroxyde d'aluminium est l'adjuvant le plus couramment utilisé. Ces sels minéraux utilisés comme adjuvants de compositions vaccinales sont décrits notamment dans l'article de Rajesh K. Gupta et al "Adjuvants, balance between toxicity and adjuvanticity", Vaccine, vol. 11, Issue 3, 1993, pages 993-306.

Les adjuvants mentionnés ci-dessus présentent comme inconvénient une efficacité faible. En outre, il est connu que l'hydroxyde d'aluminium n'induit efficacement qu'une immunité humorale, et non une immunité cellulaire. Par ailleurs, ils peuvent induire une certaine toxicité vis-à-vis des sujets traités. Plus particulièrement, lorsque ces compositions vaccinales sont injectées aux sujets à vacciner, on peut observer la formation de lésions et d'autres réactions locales telles que des granulomes au niveau du point d'injection.

D'autres adjuvants, constitués de sels de métaux divalents ou trivalents ou encore de composés sympathomimétiques sont décrits respectivement dans les demandes internationales de brevet publiées sous les numéros WO 96/32964, WO 98/17311 et WO 98/15288.

Les émulsions sont des mélanges stables constitués par un système de deux liquides non miscibles dont l'un est divisé en gouttelettes dans l'autre. Ainsi, les émulsions comprennent des huiles, une phase aqueuse et des agents tensioactifs qui ont pour fonction de disperser l'une des deux phases dans l'autre et d'obtenir un mélange macroscopiquement homogène et stable. Elles sont souvent utilisées comme adjuvants de l'immunité dans les formulations vaccinales.

L'utilisation d'huiles minérales comme adjuvant de vaccin est connues depuis de nombreuses années (Freund 1956, Herbert 1967). Les huiles minérales utilisées comme adjuvants de vaccins sont des hydrocarbures liquides, qui sont obtenues par distillation du pétrole et par la mise en oeuvre d'étapes de traitement subséquentes comme par exemple les étapes de désulfurisation, de désasphaltage, d'extraction des composés aromatiques, d'extraction des cires, et autres étapes de traitement de finition. Les études de l'influence de la qualité des huiles sur la réponse immunitaire n'est venue que progressivement et de manière incomplète. En effet, la composition des huiles minérales est très influencée par l'origine géographique des bruts utilisés ainsi que du procédé (chimie et équipement) mis en oeuvre pour leur préparation. Les études effectuées sur les différentes fractions étaient destinées à évaluer le caractère toxique pour les travailleurs utilisateurs mais pas pour des utilisations en santé animale ou humaine). Les optimisations de composition de ces huiles minérales, basées sur des caractérisations macroscopiques (viscosité, point de fusion) ont été effectuées avec des grades d'huiles minérales industrielles existantes, ou de produits chimiques purs de laboratoires ne correspondant pas à des définitions ni à la caractérisation de grades de matières premières acceptables. Ainsi, les études même poussées, effectuées sur des grades industriels d'huiles minérales définies par des paramètres ne reflétant par leurs propriétés biologiques entraînaient et entraînent encore des variations de comportements lors de l'utilisation pour des vaccins en fonction de l'origine d'un lot d'huile minérale. Ces lots, bien que répondant à leur cahier des charges et spécifications analytiques macroscopiques et portant le même nom commercial, ont des compositions suffisamment différentes pour ne pas donner les mêmes propriétés aux vaccins. Par ailleurs, la viscosité des émulsions obtenues à partir des huiles minérales, sans optimisation galénique, induisait un biais dans l'interprétation des résultats.

L'influence de la répartition entre chaînes linéaires et cyclisée a été évaluée pour rechercher une éventuelle toxicité lors de l'utilisation des huiles minérales en injectable suspectant le rôle tératogène des formes cyclisées. Les critères de toxicités ont ensuite été identifiés comme provenant des formes insaturées, éliminées parfaitement dans les procédés actuels par hydrogénation poussée.

Aucune étude n'a été effectuée dans le but d'évaluer l'influence du ratio de constituants de type linéaires ou cyclisés sur la qualité de la réponse immunitaire.

Les huiles minérales ont été utilisées depuis plusieurs décennies comme adjuvants et pour la préparation d'adjuvants destinés à augmenter l'efficacité de la réponse immunitaire des vaccins. En effet, des antigènes préalablement émulsionnés dans des émulsions de type eau dans huile minérale (E/H) puis administrés par voie parentérale déclenchent des réponses immunitaires (et par conséquence des protections contre les pathogènes) beaucoup plus intenses et durables.

La nature de l'huile minérale utilisée pour la préparation d'adjuvant a une influence importante sur l'intensité de la réponse immunologique et sur les réactions secondaires liées à l'injection du vaccin (réactions locales et générales) (J. Aucouturier et al., "Montanide ISA 720 and 51: a new génération of water in oil émulsions as adjuvants for human vaccines", Expert review of vaccines, vol:1, 2002, pages 111-118).

Il y a deux types de réponses immunitaires :
- La réponse immunitaire humorale.
   C'est la réaction qui se produit lorsque des lymphocytes B possédant des récepteurs spécifiques sont stimulés par un antigène et se différencient en clone de plasmocytes qui commencent à sécréter des anticorps. Ceux-ci sont efficaces contre les agents pathogènes circulant dans le sang et la lymphe. De plus, l'activation sélective des lymphocytes B dote l'organisme de cellules mémoires à durée de vie prolongée qui interviennent dans la réponse immunitaire secondaire.
- La réponse immunitaire cellulaire.

La réaction immunitaire humorale aide le réseau de défense à reconnaître et détruire les agents pathogènes libres, mais c'est la réaction à médiation cellulaire qui combat les agents pathogènes déjà introduits dans les cellules. Les principaux acteurs de l'immunité à médiation cellulaire sont les lymphocytes T.

Les lymphocytes T ne réagissent qu'aux déterminants antigéniques exposés à la surface des cellules de l'organisme. Les lymphocytes T reconnaissent ces déterminants grâce à leurs récepteurs, des protéines surfaciques enchâssées dans leur membrane plasmique. Le récepteur du lymphocyte T reconnaît l'Antigène combiné avec une des glycoprotéines du CMH de l'organisme. Outre leur rôle dans la réponse humorale, les Lymphocytes T auxiliaires peuvent également activer d'autres types de lymphocytes T pour déclencher les réactions à médiation cellulaire contre des Antigènes. Les Lymphocytes T cytotoxiques sont les seules cellules à tuer d'autres cellules (celles infectées par des agents pathogènes intra cellulaires).

Les adjuvants huileux sont très connus pour augmenter la composante humorale de la réponse immunologique (mesurée en général par des dosages d'immunoglobulines du groupe 1 ou IGG1) alors que des réponses de type cellulaires (mesurées indirectement par les IGG2) sont très difficiles à obtenir.

Il est connu que les émulsions E/H, donnant les plus fortes réponses immunitaires, induisent aussi une composante de type cellulaire mais dans des proportions moindres par exemples que certaines molécules solubles (type saponines ou tensioactifs cationiques) réputées pour orienter la réponse vers des mécanismes cellulaires.

Les émulsions à phase continue aqueuse utilisant les huiles minérales classiquement sur le marché, sont de très mauvais promoteurs de la réponse cellulaire.

En effet, le type de réponse engendrée par les vaccins est influencé par la structure de l'antigène, mais également par la façon dont celui ci est présenté au système immunitaire via les adjuvants. Les adjuvants ont différents effets sur la réponse immunitaire, car ils peuvent induire une réponse immunitaire sur une durée plus ou moins longue, avec un caractère prédominant de type cellulaire ou de type humoral.

L'immunité à médiation cellulaire est importante, voire parfois essentielle pour la protection contre les bactéries intracellulaires, les virus et la plupart des parasites. Elle intervient dans la plupart des mécanismes de protection vaccinale en complément de la réponse humorale.

On pourra par exemple classer la capacité d'un adjuvant à induire une réponse immunologique de type cellulaire pour la composition vaccinale le comprenant e n comparant les rapports IGG1/IGG2 mesurés suite à l'administration par voie parentérale de la composition vaccinale le comprenant aux sujets concernés, à des périodes différentes suivant le jour de vaccination desdits sujets. Plus ce rapport sera élevé et plus l'adjuvant aura orienté la réponse immunitaire de la composition vaccinale vers une composante humorale. Une valeur du rapport IGG1/IGG2 proche de l'unité sera considérée comme le signe d'une réponse immunitaire de la composition vaccinale équilibrée entre ses deux composantes. Une valeur du rapport IGG1/IGG2 inférieure à l'unité sera caractéristique d'une réponse immunitaire de la composition vaccinale à prédominance cellulaire.

Un but de la présente invention est de disposer d'un adjuvant pour la préparation de compositions vaccinales, destiné à améliorer la composante cellulaire de la réponse immunitaire, tout en maintenant le bon niveau de la composante humorale. Il y a donc un besoin d'obtenir des adjuvants de vaccins ayant une réponse immunitaire équilibrée voire à prédominance cellulaire. En outre les vaccins préparés avec l'adjuvant objet de la présente invention devront présenter une bonne innocuité.

A cette fin, la présente invention, a pour objet un adjuvant de vaccin comprenant pour 100% de sa masse :
- de 10 % à 95 % d'une huile minérale comprenant :
   - de 0,05% massique à 10% massique de chaînes hydrocarbonées ayant moins de 16 atomes de carbone ;
   - de 0,05% massique à 5% massique de chaînes hydrocarbonées ayant plus de 28 atomes de carbone ;
et possédant un rapport P/N, correspondant au rapport de la quantité massique des chaînes hydrocarbonées de type paraffinique sur la quantité massique des chaînes hydrocarbonées de type naphténique, compris entre 2,8 et 2,9, de préférence égal à 2,85.

De préférence, ledit adjuvant comprend de 20% à 90% de ladite huile minérale.

Par ailleurs, des modes de réalisation de l'invention peuvent comporter l'une ou plusieurs des caractéristiques suivantes :
- Adjuvant tel que défini ci-dessus caractérisé en ce qu'il comprend en outre :
   - de 5 % à 90 % d'au moins un tensioactif. De préférence de 5% à 50% de tensioactif.
- Adjuvant tel que défini ci-dessus caractérisé en ce qu'il est sous forme d'une émulsion huile dans eau (H/E) ou eau dans huile dans eau (E/H/E).
- Adjuvant tel que défini ci-dessus caractérisé en ce qu'il est sous forme d'une émulsion eau dans huile (E/H).

Selon un autre aspect, l'invention a pour objet un vaccin comprenant l'adjuvant tel que défini ci-dessus ainsi qu'au moins un antigène ou au moins un générateur in vivo d'un composé comprenant une séquence d'acides aminés.

Par ailleurs, des modes de réalisation de l'invention peuvent comporter l'une ou plusieurs des caractéristiques suivantes :
Vaccin tel que défini ci-dessus, caractérisé en ce qu'il est sous forme d'émulsion du type eau dans huile (E/H) ou huile dans eau (H/E) ou eau dans huile dans eau (E/H/E).

Selon un autre aspect de l'invention, celle-ci consiste en l'utilisation d'une huile minérale pour la fabrication d'un adjuvant de vaccin tel que défini ci-dessus, caractérisée en ce que la dite huile comprend pour 100% de sa masse :
- de 0,05% à 10% de chaînes hydrocarbonées ayant moins de 16 atomes de carbone ;
- de 0,05% à 5% de chaînes hydrocarbonées ayant plus de 28 atomes de carbone ;
et possède un rapport P/N, correspondant au rapport de la quantité massique de chaînes carbonées de type paraffinique sur la quantité massique de chaînes carbonées de type naphténique, compris entre 2,8 et 2,9, de préférence égal à 2,85.

Selon un autre aspect de l'invention, celle-ci consiste en l'utilisation d'une huile minérale pour la fabrication d'un vaccin tel que défini à l'une des ci-dessus, caractérisée en ce que la dite huile comprend :
- de 0,05% à 10% de chaînes hydrocarbonées ayant moins de 16 atomes de carbone ;
- de 0,05% à 5% de chaînes hydrocarbonées ayant plus de 28 atomes de carbone ;
et possède un rapport P/N, correspondant au rapport de la quantité massique de chaînes carbonées de type paraffinique sur la quantité massique de chaînes carbonées de type naphténique, compris entre 2,8 et 2,9, de préférence égal à 2,85.

Selon un autre aspect de l'invention, celle-ci consiste en l'utilisation d'un adjuvant tel que défini ci-dessus pour la préparation d'un vaccin destiné à la prévention ou au traitement d'une maladie infectieuse.

Selon un autre aspect de l'invention, celle-ci consiste en l'utilisation de cet adjuvant pour la préparation d'une composition destinée à soigner une maladie d'ordre fonctionnel, telle le cancer ou la mucoviscidose.

La composition selon l'invention peut également avantageusement comporter un ou plusieurs agents tensioactifs émulgateurs. Ce dernier présente un caractère lipophile ou hydrophile caractérisé par une valeur HLB (hydrophile-lipophile-balance) comprise entre 1 et 19.

De préférence les tensioactifs sont choisis parmi :
∘ les esters de sorbitan, comme par exemple l'oléate de sorbitan, le stéarate de sorbitan, le palmitate de sorbitan, le laurate de sorbitan ;
∘ les esters mannitan, comme par exemple l'oléate de mannitan, le stéarate de mannitan, le palmitate de mannitan, le laurate de mannitan ;
∘ les esters de sorbitan polyoxyéthylés entre 5 moles et 20 moles d'oxyde d'éthylène, comme par exemple l'oléate de sorbitan éthoxylé à 20 moles d'oxyde d'éthylène ;
∘ les esters de mannitan polyoxyéthylés entre 5 moles et 20 moles d'oxyde d'éthylène, comme par exemple l'oléate de mannitan éthoxylé à 20 moles d'oxyde d'éthylène ;
∘ les lécithines ;
∘ les alcanols polyoxyéthylés comme par exemple ceux commercialisés sous l'appellation BRIJ, et plus particulièrement le BRIJ 21 et le BRIJ 221 par la société UNIQEMA ;
∘ les polymères tensioactifs comprenant des blocs polyoxyéthylènes et polyoxypropylènes, tels que ceux commercialisés sous la désignation PLURONICS par la société BASF ;
∘ les polyhydroxystéarates de polyglycol ou de polyglycérol comme par exemple les produits dénommés HYPERMER™ B246, ARLACEL™P135 commercialisés par la société UNIQEMA.

Des tensioactifs particulièrement préférés sont les esters de polyéthylèneglycol, obtenus par condensation d'un acide gras, notamment un acide gras liquide à 20°C avec un polyéthylèneglycol de poids moléculaire compris entre 80 et 200 ; un tel tensioactif est commercialisé par la Société SEPPIC sous la marque SIMULSOL 2599.

Un autre agent tensioactif préféré dans le cadre de la présente invention consiste en un ester obtenu par condensation d'un acide gras, avantageusement un acide gras liquide à 20°C avec un sucre, le sorbitol ou du glycérol. Ledit sucre peut consister en glucose, saccharose ou, de préférence, mannitol. A titre d'ester de mannitol particulièrement préféré, on peut citer des oléates de mannitol obtenus par anhydrisation de la chaîne carbonée polyhydroxylée du mannitol qui se cyclise en 1-4 ou en 2-6.

Des dérivés de ces esters de sucre de polyéthylèneglycol, de sorbitol ou de glycérol peuvent être également mis en oeuvre. Ces dérivés présentent une hydrophilie modifiée notamment par greffage de fonctions hydrophiles telles que alcool, polyol, oxyde d'éthylène, oxyde de propylène, acide carboxylique, amine ou amide. De tels dérivés peuvent par exemple consister en des esters gras de sorbitanne polyoxyéthylés, tels le Montanox™80.

Les autres types de tensioactifs préférés consistent en des huiles végétales éthoxylés, telles que, par exemple l'huile de ricin polyéthoxylée, cette huile étant hydrogénée ou non, ou des esters de polyglycerol, notamment des esters de polyglycérol d'acides gras naturels tels que l'acide oléique, l'acide stéarique, l'acide ricinoléique ou l'acide isostéarique et, particulièrement, des ricinoléates ou des polyricinoléates de polyglycérol.

Un agent tensioactif selon l'invention est de préférence pharmaceutiquement acceptable au niveau des muqueuses ; il doit notamment être dépourvu de métaux lourds et présenter des indices d'acides ou de peroxydes très faibles. Il est également souhaitable qu'il satisfasse les normes de tests d'innocuité tels que par exemple, ceux décrits par S.S. Berllin, Annales of Allergy, 1962, 20, 473 ou les tests de toxicité anormale décrits dans la pharmacopée européenne. Préférentiellement, l'agent tensioactif est associé à l'adjuvant huileux avant formation de l'émulsion.

Par antigène ou au moins un générateur in vivo d'un composé comprenant une séquence d'acides aminés, on désigne soit des micro-organismes tués, tels que les virus, les bactéries ou les parasites, soit des fractions purifiées de ces micro-organismes, soit des micro-organismes vivants dont le pouvoir pathogène a été atténué. On peut citer notamment un virus recombinant notamment un virus non enveloppé tel que les adénovirus, le virus de la vaccine, le virus Canarypox, les herpès virus, les baculovirus. On désigne aussi un vecteur recombinant viral non enveloppé vivant dont le génome contient, insérée de préférence dans une partie non essentielle pour la réplication du virus enveloppé correspondant, une séquence codant pour une sous-unité antigénique induisant une synthèse d'anticorps et/ou un effet protecteur contre le susdit virus enveloppé ou micro-organisme pathogène. Ces sous-unités antigéniques peuvent être par exemple, une protéine, une glycoprotéine, un peptide, ou une fraction peptidique et/ou protectrice contre une infection par un micro-organisme vivant tel un virus enveloppé, une bactérie ou un parasite.

On peut citer notamment un plasmide recombinant constitué d'une séquence de nucléotide dans laquelle est insérée une séquence nucléotidique exogène, provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte.

Les huiles minérales sont obtenues à partir de la distillation puis de l'hydrogénation poussée de produits d'origine pétrolière. Elles sont constituées par des molécules chimiques de la famille des alcanes saturés. Pour ce type de molécules, le nombre d'atomes de carbone (n) est en général supérieur à 9 et inférieur à 30, conférant alors un aspect liquide à 25°C. On parle aussi de paraffines liquides. Les paraffines solides sont constituées par des molécules chimiques de la famille des alcanes pour lesquelles n est supérieur à 30, pouvant aller jusqu'à 50 et confèrent alors un aspect solides à 25°C. Les paraffines liquides, ou huiles minérales, sont des liquides transparents et inodores, dont la viscosité dynamique peut varier de 2 milliPascal.seconde (mPa.s) à 1 Pascal.seconde. La viscosité dynamique des huiles minérales est directement liée à la longueur moyenne des chaînes carbonées des alcanes composant lesdites huiles minérales. Les alcanes à chaînes hydrocarbonées les plus longues sont responsables des viscosités dynamiques les plus élevées. Les molécules concernées peuvent se présenter sous forme de chaînes linéaires hydrocarbonées ou linéaires branchées (regroupées sous la dénomination « paraffinique » (P) ou cyclisées (naphténiques N)) mais toujours saturées (sans doubles liaisons qui sont éliminées lors du traitement par l'hydrogénation). Les viscosités, points de fusion, et points d'ébullition sont directement liés au nombre de carbone que présente la chaîne hydrocarbonée. Une huile blanche minérale pourra donc être caractérisée par son intervalle de distillation, sa viscosité dynamique, son nombre d'atomes de carbone moyen, mais aussi par sa fraction P/N en molécules linéaires et branchées sur la quantité des molécules cyclisées (déterminée selon la méthode DIN 51378). A noter que le critère de viscosité dynamique peut être modifié artificiellement par mélange de deux coupes différentes.

Les exemples suivants permettent de mettre en évidence les propriétés des compositions adjuvantes selon la présente invention.

Dans ce travail, une étude des propriétés adjuvantes de différentes huiles caractérisées finement à permis de mettre en évidence des compositions originales nouvelles caractérisées par des limites de compositions établies par chromatographie.

L'étude effectuée sur des compositions vaccinales expérimentales dans un premier temps avec des formules se présentant sous la forme d'émulsions Eau-dans-huile (E/H) à permis d'établir les conditions optimales d'obtention de réponses immunitaires équilibrées entre ses deux composantes et d'une réponse immunitaire à composante cellulaire prédominante pour des formules bien tolérées sur des souris. La suite des essais expérimentaux, réalisés sur d'autres types de formules et différents animaux a permis de mettre en évidence que les compositions adjuvantes objets de la présente invention assurent les meilleures réponses immunologiques tant quantitatives que qualitatives.

Dans les exemples qui suivent, les huiles minérales sélectionnées sont caractérisées par leur:
- Fraction courte (FC) exprimé en pourcent, correspondant à la proportion d'entités ayant un nombre d'atomes de carbone C < 16.
- Fraction lourde (FL) exprimé en pourcent, correspondant à la proportion d'entités ayant un nombre d'atomes de carbone C > 28.
- Répartition moléculaire (RM) (exprimée en nombre d'atomes de carbone C) calculée comme le barycentre de la courbe comprise entre C16 et C28 en attribuant le coefficient surfacique des fractions C16 à C20 assimilées à 18 atomes de carbone, 20 atomes de carbone à 24 atomes de carbone assimilées à 22 atomes de carbone et 24 atomes de carbone à C28 assimilées à 26 C.
- le rapport P/N (obtenu selon la méthode normalisée DIN 51378).

Les différentes huiles testées sont listées dans le tableau 1.

**Tableau 1 : panel d'huiles synthétisées et caractérisées pour essais biologiques.**

| **Ref Huile** | **FC (<C16)** | **RM** | **FL (>C28)** | **P/N** |
|---|---|---|---|---|
| 1 | 13.7 | 20.6 | 3.3 | 1.78 |
| 2 | 4.9 | 21.5 | 7.1 | 1.86 |
| 3 | 4.2 | 20.5 | 1.5 | 1.86 |
| 4 | 4.4 | 21.6 | 4.7 | 1.86 |
| 5 | 47.7 | 18.4 | 0.5 | 1.86 |
| 6 | 0.3 | 22.2 | 9.6 | 2.03 |
| 7 | 30 | 18.3 | 0.6 | 2.19 |
| 8 | 16 | 21.8 | 10 | 2.13 |
| 9 | 2 | 19.3 | 2.2 | 2.85 |
| 10 | 48.2 | 18.4 | 0.6 | 1.86 |
| 11 | 8.6 | 20.7 | 2.4 | 1.94 |
| 12 | 20.8 | 21.9 | 8.7 | 1.86 |

### ETUDE SOUS LA FORME D'EMULSION EAU DANS HUILE E/H :

La formulation est conduite dans un modèle type adjuvant incomplet de Freund (IFA) utilisé à 50% dans l'émulsion vaccinale qui par la simplicité de sa formule (mannide monooléate 15% + huile minérale 85%) permet une comparaison pertinente.

Les propriétés biologiques des vaccins ainsi formulés (50% IFA + 50% de solution isotonique d'albumine d'oeuf à 10 µg / dose ; 1 dose = 100 µl d'émulsion) ont alors été comparées sur modèle souris pour les paramètres d'innocuité (tableau 2), et d'efficacité pour la réponse humorale ou cellulaire.

L'innocuité est évaluée par observation du site d'injection 7 jours après vaccination puis notation des réactions locales (RL) sur une échelle allant de 0 à 4. Les valeurs de 0 à 2 sont acceptables (il s'agit au maximum de dépôt huileux sans alopécie, pas d'induration palpable ni site d'injection visible) ; les valeurs supérieures à 2 sont considérées comme non souhaitées et inacceptables (il s'agit de nécroses, alopécies, induration palpables). Les résultats sont groupés dans le tableau 2 ; les huiles minérales testées dans la préparation de la formulation E/H ont été classées en fonction de leur FC. Il apparaît très clairement que seules les formulations E/H comprenant des huiles minérales ayant un FC < à 13,7 sont appropriées pour la préparation de compositions vaccinales utilisables en injectable.

**Tableau 2 : évaluation de l'innocuité des différentes huiles.**

| **Ref Huile** | **FC (<C16)** | **RM** | **FL (>C28)** | **P/N** | RL |
|---|---|---|---|---|---|
| 1 | 13.7 | 20.6 | 3.3 | 1.78 | 2.5 |
| 2 | 4.9 | 21.5 | 7.1 | 1.86 | 0.5 |
| 3 | 4.2 | 20.5 | 1.5 | 1.86 | 0 |
| 4 | 4.4 | 21.6 | 4.7 | 1.86 | 0.5 |
| 5 | 47.7 | 18.4 | 0.5 | 1.86 | 4 |
| 6 | 0.3 | 22.2 | 9.6 | 2.03 | 0 |
| 7 | 30 | 18.3 | 0.6 | 2.19 | 3 |
| 8 | 16 | 21.8 | 10 | 2.13 | 2.5 |
| 9 | 2 | 19.3 | 2.2 | 2.85 | 0 |
| 10 | 48.2 | 18.4 | 0.6 | 1.86 | 4 |
| 11 | 8.6 | 20.7 | 2.4 | 1.94 | 0.5 |
| 12 | 20.8 | 21.9 | 8.7 | 1.86 | 3 |

Les formules retenues ont ensuite été testées pour leur efficacité immunologique dans le modèle souris. Les formules donnant des réactions inacceptables ont été éliminées. Les tableaux 3 et 4 montrent les titres anticorps obtenus, comparés à une référence d'hydroxyde d'aluminium (adjuvant solide minéral utilisé en médecine vétérinaire et humaine). Les titres sont donnés à 14/28/42/56/90 jours après la date des premières injections, pour la réponse humorale (IGG1) et cellulaire (IGG2). Une seconde injection des formules est réalisée à 28 jours, après l'enregistrement des titres anticorps. Les résultats pour la réponse à long terme (90 jours) indiquent une corrélation forte entre des faibles valeurs de fraction lourde et l'intensité de la réponse humorale à 90 jours. La réponse est significativement diminuée lorsque cette fraction est supérieure à 4.7% (7.1% et 9.6%).

Ces résultats démontrent qu'une huile minérale pour adjuvant de vaccin sous forme d'émulsion E/H contenant moins de 10% de FC, moins de 5% de FL et un ratio P/N supérieur ou égal à 2 induit une bonne réponse de type cellulaire.

**Tableau 3 : Réponse anticorps de type IgG1 contre OVA dans la souris pour différentes huiles après injection sous forme émulsion E/H en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 9 600 | 16 000 | 128 000 | 128 000 | 128 000 |
| 3 | 4.2 | 20.5 | 1.5 | 1.86 | 9 600 | 16 000 | 128 000 | 256 000 | 256 000 |
| 4 | 4.4 | 21.6 | 4.7 | 1.86 | 9 600 | 16 000 | 128 000 | 256 000 | 256 000 |
| 6 | 0.3 | 22.2 | 9.6 | 2.03 | 9 600 | 16 000 | 64 000 | 64 000 | 64 000 |
| 9 | 2 | 19.3 | 2.2 | 2.85 | 9 600 | 16 000 | 128 000 | 256 000 | 256 000 |
| 11 | 8.6 | 20.7 | 2.4 | 1.94 | 9 600 | 16 000 | 128 000 | 256 000 | 256 000 |
| AIOH | | | | | 16 000 | 4 800 | 4 800 | 2 400 | 2 400 |

**Tableau 4 : Réponse anticorps de type IgG2 contre OVA dans la souris pour différentes huiles après injection sous forme émulsion E/H.**

| **Ref Huile** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 9600 | 16 000 | 48 000 | 48000 | 48 000 |
| 3 | 4.2 | 20.5 | 1.5 | 1.86 | 9600 | 16 000 | 48000 | 48 000 | 48 000 |
| 4 | 4.4 | 21.6 | 4.7 | 1.86 | 9600 | 16 000 | 48000 | 48000 | 48 000 |
| 6 | 0.3 | 22.2 | 9.6 | 2.03 | 9600 | 16 000 | 128 000 | 256 000 | 256 000 |
| 9 | 2 | 19.3 | 2.2 | 2.85 | 9600 | 16 000 | 128 000 | 256 000 | 256 000 |
| 11 | 8.6 | 20.7 | 2.4 | 1.94 | 9600 | 16 000 | 128 000 | 256 000 | 256 000 |
| AIOH | | | | | 16 000 | 4 800 | 4 800 | 2400 | 2400 |

### ETUDE SOUS LA FORME D'EMULSION A PHASE CONTINUE AQUEUSE :

Deux types galéniques représentant les émulsions à phase continue aqueuse (multiple E/H/E et microémulsion H/E) ont été testés.

### Microémulsion Huile dans eau :

La formule adjuvante comprend pour 100% de sa masse 40% massique d'un tensioactif hydrophile (le polysorbate 80) et 60% massique d'huile minérale à tester ; la phase huileuse ainsi formulée est émulsionnée à température ambiante par un homogénéisateur à haute pression en présence d'une phase aqueuse comprenant l'antigène (OVA) ; la phase huileuse précédemment préparée est utilisée à 10% massique dans la composition vaccinale finale (OVA 10 µg/dose, 100 µl par injection); un témoin sur hydroxyde d'aluminium est inclus dans l'essai. Les résultats sont présentés dans le tableau 5. La faible intensité de la réponse IGG1 est directement corrélée avec la présence de FL, qui inhibe la réponse à court terme (28 jours, J28) et à long terme (90 jours, J90).

De même, le tableau 6 présente les titres en IGG2.

Les résultats indiquent une corrélation entre le rapport P/N et la réponse IGG2 ; les formules 6 et 9 se démarquant des autres produits.

**Tableau 5 : Réponse anticorps de type IgG1 contre OVA dans la souris pour différentes huiles sous forme microémulsion H/E en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.9 | 12 800 | 16 000 | 96 000 | 48 000 | 48 000 |
| 3 | 4.2 | 20.5 | 1.5 | 1.9 | 9 600 | 48 000 | 128 000 | 256 000 | 256 000 |
| 4 | 4.4 | 21.6 | 4.7 | 1.9 | 12 800 | 48 000 | 128 000 | 128 000 | 128 000 |
| 6 | 0.3 | 22.2 | 9.6 | 2.0 | 4 800 | 16 000 | 48 000 | 48 000 | 32 000 |
| 9 | 2.0 | 19.3 | 2.2 | 2.8 | 6 400 | 48 000 | 32 000 | 128 000 | 128 000 |
| 11 | 8.6 | 20.7 | 2.4 | 1.9 | 9 600 | 48 000 | 128 000 | 256 000 | 256 000 |
| AIOH | | | | | 16 000 | 4 800 | 4 800 | 2 400 | 2 400 |

**Tableau 6 : Réponse anticorps de type IgG2 contre OVA dans la souris pour différentes huiles sous forme microémulsion H/E en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 400 | 12000 | 24 000 | 4 000 | 400 |
| 3 | 4.2 | 20.5 | 1.5 | 1.86 | 600 | 16000 | 24 000 | 1 000 | 1000 |
| 4 | 4.4 | 21.6 | 4.7 | 1.86 | 150 | 8000 | 8 000 | 1 000 | 1000 |
| 6 | 0.3 | 22.2 | 9.6 | 2.03 | 4800 | 16000 | 32 000 | 8 000 | 4000 |
| 9 | 2 | 19.3 | 2.2 | 2.85 | 1600 | 32000 | 48 000 | 96 000 | 96000 |
| 11 | 8.6 | 20.7 | 2.4 | 1.94 | 600 | 16000 | 24 000 | 1 000 | 4000 |
| AlOH | | | | | 150 | 150 | 150 | 300 | 300 |

Les comparaisons des ratios IgG1/IgG2 indiquent clairement une différence de comportement de la réponse pour les groupes vaccinés avec l'huile 9 et à moindre niveau avec l'huile 6 par rapport aux autres huiles (tableau 7).

**Tableau 7 : Ratio IgG1/IgG2 contre OVA dans la souris pour différentes huiles sous forme microémulsion H/E en fonction du temps.**

| **HUILE REF** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 32 | 1 | 4 | 12 | 120 |
| 3 | 4.2 | 20.5 | 1.5 | 1.86 | 16 | 3 | 5 | 256 | 256 |
| 4 | 4.4 | 21.6 | 4.7 | 1.86 | 85 | 6 | 16 | 128 | 128 |
| 6 | 0.3 | 22.2 | 9.6 | 2.03 | 1 | 1 | 2 | 6 | 8 |
| 9 | 2 | 19.3 | 2.2 | 2.85 | 4 | 2 | 1 | 1 | 1 |
| 11 | 8.6 | 20.7 | 2.4 | 1.94 | 16 | 3 | 5 | 256 | 64 |
| AlOH | | | | | 107 | 32 | 32 | 8 | 8 |

### Emulsion multiple Eau-dans-Huile-Eau (E/H/E) :

La formule adjuvante est cette fois composée d'un anhydromannitol octadecenoate éther de balance hydrophile lipophile (HLB) = 9, dispersé à 15% massique dans l'huile minérale à tester. La composition vaccinale est préparée en mélangeant à 30°C une part en poids d'adjuvant avec une part en poids de milieu antigénique (phase aqueuse) comprenant l'antigène OVA.
Essai sur souris : les résultats de l'essai souris pour les réponses IGG1 et pour les réponses IGG2 sont présentés respectivement dans les tableaux 8 et 9. En ce qui concerne la réponse cellulaire, l'évolution des valeurs des réponses IGG2 indique que seule l'huile 9 induit une réponse intense et durable, notamment après 28 jours.
Essai sur modèle bovin : dans ce cas, les mêmes vaccins ont été testés sur 5 bovins injectés avec 2 ml en sous-cutané; les formules 9 et 11 ont été comparées. Les résultats sont présentés dans le tableau 10. Aucune différence n'est constatée pour les titres IGG1 (données non présentées). Un fort effet adjuvant est observé par rapport à l'antigène seul. La réponse IGG2 est très intense avec l'huile 9, faible avec l'huile 11 et inexistante pour l'antigène seul.

**Tableau 8 : Réponse anticorps de type IgG1 contre OVA dans la souris pour différentes huiles sous forme émulsion E/H/E en fonction du temps.**

| **Bref Huile** | **FC (<C16)** | **RM** | **FL (>C28)** | **P/N** | **J14** | **J28** | **J42** | **J56** | **J90** |
|---|---|---|---|---|---|---|---|---|---|
| 2.0 | 4.9 | 21.5 | 7.1 | 1.9 | 16000 | 32000 | 64000 | 64000 | 64000 |
| 3.0 | 4.2 | 20.5 | 1.5 | 1.9 | 16000 | 64000 | 128000 | 128000 | 128000 |
| 4.0 | 4.4 | 21.6 | 4.7 | 1.9 | 16000 | 64000 | 128000 | 128000 | 128000 |
| 6.0 | 0.3 | 22.2 | 9.6 | 2.0 | 16 000 | 64000 | 64 000 | 64 000 | 64000 |
| 9.0 | 2.0 | 19.3 | 2.2 | 2.8 | 16 000 | 64000 | 128000 | 128000 | 128000 |
| 11.0 | 8.6 | 20.7 | 2.4 | 1.9 | 16 000 | 64000 | 128000 | 128000 | 128000 |
| AlOH | | | | | 6 000 | 4 800 | 4 800 | 2400 | 2400 |

**Tableau 9 : Réponse anticorps de type IgG2 contre OVA dans la souris pour différentes huiles sous forme émulsion E/H/E en fonction du temps.**

| **Ref Huile** | **FC (<C16)** | **RM** | **FL (>C28)** | **P/N** | **J14** | **J28** | **J42** | **J56** | **J90** |
|---|---|---|---|---|---|---|---|---|---|
| 2 | 4.9 | 21.5 | 7.1 | 1.86 | 400 | 2 000 | 24 000 | 4000 | 400 |
| 3 | 4.2 | 20.5 | 1.5 | 1.86 | 400 | 12 000 | 24 000 | 4000 | 400 |
| 4 | 4.4 | 21.6 | 4.7 | 1.86 | 400 | 12 000 | 24 000 | 4000 | 400 |
| 6 | 0.3 | 22.2 | 9.6 | 2.03 | 400 | 4 000 | 12 000 | 4000 | 400 |
| 9 | 2 | 19.3 | 2.2 | 2.85 | 9600 | 16 000 | 128 000 | 256000 | 256000 |
| 11 | 8.6 | 20.7 | 2.4 | 1.94 | 300 | 600 | 1 200 | 1200 | 1200 |
| AlOH | | | | | 150 | 150 | 150 | 300 | 300 |

**Tableau 10 : Réponse anticorps de type IgG2A contre OVA dans le bovin pour les huiles 9 et 11, sous forme émulsion E/H/E en fonction du temps.**

| Ref Huile | FC (<C16) | RM | FL (>C2 8) | P/N | J0 | J28 | J60 | J140 |
|---|---|---|---|---|---|---|---|---|
| 9 | 2 | 19.3 | 2.2 | 2.85 | - | 600 | 6000 | 10 000 |
| 11 | 8.6 | 20.7 | 2.4 | 1.94 | - | 10 | 600 | 2000 |

Par améliorer la composante cellulaire de la réponse immunitaire, on entend :
- A) obtenir une réponse immunitaire se caractérisant par un ratio IgG1/IgG2 compris entre 0,25 et 4, et
- B) obtenir une réponse immunitaire dont la composante cellulaire est aussi intense, à savoir caractérisée par un IgG2 supérieur ou égal à 32 000 après le rappel de vaccination pratiqué après 28 jours et après la mesure du titre IGG2 à cette date (réponse secondaire et long terme).

### A- ratio IgG1/IgG2

a) cas des adjuvants vaccins se présentant sous la forme d'une émulsion E/H/E, les résultats expérimentaux IgG1/IgG2 sont les suivants :
   - pour l'huile 6 : 40 (J14), 16 (J28), 5,3 (J42), 16 (J56) et 160 (J90).
   - pour l'huile 11 : 53.3 (J14), 107 (J28), 107 (J42), 107 (J56) et 107 (J90).
   - pour l'huile 9 : 1.7 (J14), 4 (J28), 1 (J42), 0.5 (J56) et 0.5 (J90).
   Seule l'huile 9 permet d'obtenir un ratio IgG1/IgG2 compris entre 0,25 et 4 avant et après le rappel de vaccination à J28 (le rappel de vaccination réalisé à J28, est effectué après la mesure de la réponse immunitaire).
b) cas des adjuvants vaccins se présentant sous la forme d'une microémulsion H/E, la déclaration d'invention comprend les résultats expérimentaux IgG1/IgG2 suivant :
   ▪ pour l'huile 6 : 1 (J14), 1 (J28), 2 (J42), 6 (J56) et 8 (J90)
   ▪ pour l'huile 11 : 16 (J14), 3 (J28), 5 (J42), 256 (J56) et 64 (J90)
   ▪ pour l'huile 9 : 4 (J14), 2 (J28), 1 (J42), 1 (J56) et 1 (J90)
   Pour l'huile 9, le ratio IgG1/IgG2 compris entre 0,25 et 4 est toujours obtenu avant le rappel de vaccination à J28, et reste constant dans le temps.
   Pour l'huile 6, le ratio IgG1/IgG2 compris entre 0,25 et 4 est aussi obtenu avant le rappel de vaccination (réponse primaire) mais ne subsiste pas dans le temps lors de la mesure de la réponse secondaire (après le rappel de vaccination de J28) et de la réponse à long terme (J90).
   Pour l'huile 11, le ratio IgG1/IgG2 compris entre 0,25 et 4 n'est pas observé pour la réponse secondaire ni pour la réponse à long terme.
c) cas des adjuvants vaccins se présentant sous la forme d'une émulsion E/H, la déclaration d'invention comprend les résultats expérimentaux IgG1/IgG2 suivant:
   ▪ pour l'huile 9 : 1 (J14), 1 (J28), 1 (J42), 1 (J56) et 1 (J90)
Tous ces résultats enregistrés montrent de bons résultats pour le ratio IgG1/IgG2.

### B- Intensité des réponses IgG2.

a) cas des adjuvants vaccins se présentant sous la forme d'une émulsion E/H/E, les résultats expérimentaux IgG2 sont les suivants (tableau 9):
   a. pour l'huile 6 (J14), 4 000 (J28), 12 000 (J42), 4 000 (J56) et 400 (J90)
   b. pour l'huile 11: 300 (J14), 600 (J28), 1200 (J42), 1200 (J56) et 1200 (J90)
   c. pour l'huile 9 : 9600 (J14), 16 000 (J28), 128 000 (J42), 256 000 (J56) et 256 000 (J90)
   Seule l'huile 9 permet d'avoir une réponse cellulaire intense (selon les critères définis précédemment) après le rappel de vaccination pratiqué après 28 jours.
b) cas des adjuvants vaccins se présentant sous la forme d'une microémulsion H/E, la déclaration d'invention comprend les résultats expérimentaux IgG2 suivant (tableau 6):
   a. pour l'huile 6 : 4800 (J14), 16 000 (J28), 32 000 (J42), 8 000 (J56) et 4000 (J90)
   b. pour l'huile 11: 600 (J14), 16 000 (J28), 24 000 (J42), 1000 (J56) et 40000 (J90)
   c. pour l'huile 9: 1600 (J14), 32 000 (J28), 48 000 (J42), 96 000 (J56) et 96 000 (J90)
   Seule l'huile 9 permet d'avoir une réponse cellulaire intense (selon les critères définis précédemment) pour la réponse secondaire (après le rappel de vaccination) et pour la réponse à long terme (J90).
c) cas des adjuvants vaccins se présentant sous la forme d'une émulsion E/H, la déclaration d'invention comprend les résultats expérimentaux IgG2 suivant (tableau 4):
   a. *pour* l'huile 9: 9600 (J14), 16 000 (J28), 128 000 (J42), 256 000 (J56) et 256 000 (J90)
Tous ces résultats enregistrés montrent de bons résultats pour la réponse IgG2.

D'après ces résultats il est bien montré qu'une composition d'une huile minérale destinée à être utilisée comme adjuvant de vaccin injectable, objet de la présente invention, comme l'huile 9 par exemple, présentant les caractéristiques suivantes :
- FC < 10% et FL < 5% ;
- P/N égal à 2,85 environ;
garantit une bonne innocuité, une bonne efficacité quant à la réponse humorale et une forte réponse cellulaire à la fois sous forme d'émulsion E/H et E/H/E et microémulsion H/E.

## Revendications

1. Adjuvant de vaccin comprenant pour 100% de sa masse :
- de 10 % à 95 % d'une huile minérale comprenant :
- de 0,05% massique à 10% massique de chaînes hydrocarbonées ayant moins de 16 atomes de carbone ;
- de 0,05% massique à 5% massique de chaînes hydrocarbonées ayant plus de 28 atomes de carbone ;
et possédant un rapport P/N, correspondant au rapport de la quantité massique des chaînes hydrocarbonées de type paraffinique sur la quantité massique des chaînes hydrocarbonées de type naphténique, compris entre 2,8 et 2,9, de préférence égal à 2,85.

2. Adjuvant selon la revendication 1 **caractérisé en ce qu'**il comprend en outre :
- de 5 % à 90 % d'au moins un tensioactif.

3. Adjuvant selon l'une des revendications précédentes **caractérisé en ce qu'**il est sous forme d'une émulsion huile dans eau (H/E) ou eau dans huile dans eau (E/H/E).

4. Adjuvant selon l'une des revendications 1 ou 2 **caractérisé en ce qu'**il est sous forme d'une émulsion eau dans huile (E/H).

5. Vaccin comprenant l'adjuvant tel que défini à l'une des revendications 1 à 4 ainsi qu'au moins un antigène ou au moins un générateur in vivo d'un composé comprenant une séquence d'acides aminés.

6. Vaccin selon la revendication 5 **caractérisé en ce qu'**il est sous forme d'émulsion du type eau dans huile (E/H) ou huile dans eau (H/E) ou eau dans huile dans eau (E/H/E).

7. Utilisation d'une huile minérale pour la fabrication d'un adjuvant de vaccin tel que défini à l'une des revendications 1 à 4, **caractérisée en ce que** la dite huile comprend pour 100% de sa masse :
- de 0,05% à 10% de chaînes hydrocarbonées ayant moins de 16 atomes de carbone ;
- de 0,05% à 5% de chaînes hydrocarbonées ayant plus de 28 atomes de carbone ;
et possède un rapport P/N, correspondant au rapport de la quantité massique de chaînes carbonées de type paraffinique sur la quantité massique de chaînes carbonées de type naphténique, compris entre 2,8 et 2,9, de préférence égal à 2,85.

8. Utilisation d'une huile minérale pour la fabrication d'un vaccin tel que défini à l'une des revendications 5 ou 6, **caractérisée en ce que** la dite huile comprend :
- de 0,05% à 10% de chaînes hydrocarbonées ayant moins de 16 atomes de carbone ;
- de 0,05% à 5% de chaînes hydrocarbonées ayant plus de 28 atomes de carbone ;
et possède un rapport P/N, correspondant au rapport de la quantité massique de chaînes carbonées de type paraffinique sur la quantité massique de chaînes carbonées de type naphténique, compris entre 2,8 et 2,9, de préférence égal à 2,85.

## Patentansprüche

1. Adjuvans für Impfstoff, das pro 100 % seiner Masse Folgendes enthält:
- 10 % bis 95 % eines Mineralöls, das Folgendes enthält:
- 0,05 Masse-% bis 10 Masse-% Kohlenwasserstoffketten mit weniger als 16 Kohlenstoffatomen;
- 0,05 Masse-% bis 5 Masse-% Kohlenwasserstoffketten mit mehr als 28 Kohlenstoffatomen;
und ein P/N-Verhältnis besitzt, das dem Verhältnis des Massegehalts der Kohlenwasserstoffketten des Paraffintyps zu dem Massegehalt der Kohlenwasserstoffketten des Naphtentyps entspricht und zwischen 2,8 und 2,9 beträgt, bevorzugt gleich 2,85 ist.

2. Adjuvans nach Anspruch 1, **dadurch gekennzeichnet, dass** es ferner Folgendes enthält:
- 5 % bis 90 % wenigstens eines Tensids.

3. Adjuvans nach einem der vorhergehenden Ansprüche, **dadurch gekennzeichnet, dass** es in Form einer Öl-in-Wasser-Emulsion (O/W) oder Wasser-in-Öl-in-Wasser-Emulsion (W/O/W) vorliegt.

4. Adjuvans nach einem der Ansprüche 1 oder 2, **dadurch gekennzeichnet, dass** es in Form einer Wasser-in-Öl-Emulsion (W/O) vorliegt.

5. Impfstoff, enthaltend das Adjuvans nach einem der Ansprüche 1 bis 4 sowie wenigstens ein Antigen oder wenigstens einen *in vivo*-Erzeuger einer Verbindung, die eine Aminosäuresequenz enthält.

6. Impfstoff nach Anspruch 5, **dadurch gekennzeichnet, dass** er in Form einer Emulsion des Typs Wasser-in-Öl (W/O) oder Öl-in-Wasser (O/W) oder Wasser-in-Öl-in-Wasser (W/O/W) vorliegt.

7. Verwendung eines Mineralöls für die Herstellung eines Adjuvans für Impfstoff nach einem der Ansprüche 1 bis 4, **dadurch gekennzeichnet, dass** das Öl pro 100 % seiner Masse Folgendes enthält:
- 0,05 % bis 10 % Kohlenwasserstoffketten mit weniger als 16 Kohlenstoffatomen;
- 0,05 % bis 5 % Kohlenwasserstoffketten mit mehr als 28 Kohlenstoffatomen;
und ein P/N-Verhältnis besitzt, das dem Verhältnis des Massegehalts der Kohlenstoffketten des Paraffintyps zu dem Massegehalt der Kohlenstoffketten des Naphtentyps entspricht und zwischen 2,8 und 2,9 beträgt, bevorzugt gleich 2,85 ist.

8. Verwendung eines Mineralöls für die Herstellung eines Impfstoffs nach einem der Ansprüche 5 oder 6, **dadurch gekennzeichnet, dass** das Öl Folgendes enthält:
- 0,05 % bis 10 % Kohlenwasserstoffketten mit weniger als 16 Kohlenstoffatomen;
- 0,05 % bis 5 % Kohlenwasserstoffketten mit mehr als 28 Kohlenstoffatomen;
und ein P/N-Verhältnis besitzt, das dem Verhältnis des Massegehalts der Kohlenstoffketten des Paraffintyps zu dem Massegehalt der Kohlenstoffketten des Naphtentyps entspricht und zwischen 2,8 und 2,9 beträgt, bevorzugt gleich 2,85 ist.

## Claims

1. Vaccine adjuvant comprising, for 100 % by weight:
- from 10 % to 95 % of a mineral oil comprising:
- from 0.05 wt.% to 10 wt.% of hydrocarbon chains having less than 16 carbon atoms;
- from 0.05 wt.% to 5 wt.% of hydrocarbon chains having more than 28 carbon atoms;
and having a P:N ratio, corresponding to the ratio of the amount by weight of paraffin-type hydrocarbon chains to the amount by weight of naphthene-type hydrocarbon chains, of between 2.8 and 2.9, preferably equal to 2.85.

2. Adjuvant according to claim 1, **characterised in that** it further comprises:
- from 5 % to 90 % of at least one surfactant.

3. Adjuvant according to either of the preceding claims, **characterised in that** it is in the form of an oil-in-water (O/W) or water-in-oil (W/O) or water-in-oil-in-water (W/O/W) emulsion.

4. Adjuvant according to either claim 1 or claim 2, **characterised in that** it is in the form of a water-in-oil (W/O) emulsion.

5. Vaccine comprising the adjuvant according to any of claims 1 to 4 and at least one antigen or at least one *in vivo* generator of a compound comprising an amino acid sequence.

6. Vaccine according to claim 5, **characterised in that** it is in the form of a water-in-oil (W/O) or oil-in-water (O/W) or water-in-oil-in-water (W/O/W) emulsion.

7. Use of a mineral oil for producing a vaccine adjuvant according to any of claims 1 to 4, **characterised in that** said oil comprises, for 100 % by weight:
- from 0.05 % to 10 % of hydrocarbon chains having less than 16 carbon atoms;
- from 0.05 % to 5 % of hydrocarbon chains having more than 28 carbon atoms;
and has a P:N ratio, corresponding to the ratio of the amount by weight of paraffin-type carbon chains to the amount by weight of naphthene-type carbon chains, of between 2.8 and 2.9, preferably equal to 2.85.

8. Use of a mineral oil for producing a vaccine according to either claim 5 or claim 6, **characterised in that** said oil comprises:
- from 0.05 % to 10 % of hydrocarbon chains having less than 16 carbon atoms;
- from 0.05 % to 5 % of hydrocarbon chains having more than 28 carbon atoms;
and has a P:N ratio, corresponding to the ratio of the amount by weight of paraffin-type carbon chains to the amount by weight of naphthene-type carbon chains, of between 2.8 and 2.9, preferably equal to 2.85.
